# EUROPEAN PATENT SPECIFICATION

(11) **EP 1 610 832 B1**
(45) Date of publication and mention of the grant of the patent: **13.07.2011**
(21) Application number: 04725735.7
(22) Date of filing: 05.04.2004
(51) Int. Cl.: A61L 24/04, A61L 27/50, A61L 24/06

(54) **BONE CEMENT COMPOSITIONS COMPRISING A NON-POLYMERISABLE ORGANOIODINE COMPOUND**
ZUSAMMENSETZUNGEN FÜR KNOCHENZEMENTE ENTHALTEND EINE NICHTPOLYMERISIERBARE ORGANOIODVERBINDUNG
COMPOSITION DE CIMENT ORTHOPEDIQUE COMPRENANT UN COMPOSE ORGANOIODE NON POLYMERISABLE

(30) Priority: 04.04.2003 GB 0307834
(43) Date of publication of application: 04.01.2006
(73) Proprietor: TA Contrast AB, 23940 Falsterbo (SE); Almen, Torsten, 23940 Falsterbo (SE)
(72) Inventor: ALMEN, Torsten, SE23940 Falsterbo (SE); KLAVENESS, JO, SE23940 Falsterbo (SE); BRUDELI, Bjarne, SE23940 Falsterbo (SE)
(74) Representative: Cockbain, Julian
(86) International application number: PCT/GB2004/001509
(87) International publication number: WO 2004/087229

(56) References cited:
- EP-A- 0 676 212
- WO-A-99/62570
- US-A- 5 902 839
- US-A- 6 040 408
- VAZQUEZ B ET AL.: "Radiopaque acrylic cements prepared with a new acrylic derivative of iodo-quinoline" BIOMATERIALS, vol. 20, no. 21, November 1999 (1999-11), pages 2047-2053, XP002308104 cited in the application

## Description

This invention relates to bone cement compositions and kits therefor and to the use of organoiodine compounds in the manufacture of bone cements.

In the elderly, in particular, it is frequently necessary to replace a skeletal joint (commonly the hip joint) with a prosthetic joint. Currently, about one million such operations are carried out annually. In the case of the hip joint, a metal pin carrying a replacement "ball" for the ball-in-socket joint is cemented into the longitudinal cavity in the femur, usually using an acrylic bone cement.

Such joint prostheses often have to be repaired or replaced, e.g. as a result of the metal pin becoming loose.

In order that the placement of the metal pin within the femur may be monitored, e.g. shortly after the operation to insert the prosthesis or over the subsequent years, it is common for the bone cement used to contain a compound with the ability to absorb X-rays, i.e. a radioopaque material such as zirconia. In general, such radioopaque materials have been compounds of heavy metals, for example incorporated into the bone cement as insoluble particles. This however has the drawbacks that the particles reduce the mechanical strength of the polymer matrix of the set cement and that any release of the radioopaque material from the surface of the cement or on failure of the cement distributes highly abrasive particles into the joint.

WO 99/62570 A discloses a method for preparing a bone cement comprising a non-polymerisable organoiodine compound with polymeric particles and a polymerisable substance.

One solution that has been proposed has been to utilize as a monomer, in the preparation of one component of the bone cement polymer matrix, a compound comprising an iodophenyl group linked to an acrylic group via an ester group (e.g. 2-methacryloyloxyethyl (2,3,5-triiodobenzoate), 2-methacryloyloxypropyl(2,3,5-triiodobenzoate), and 3-methacryloyloxypropyl-1,2-bis (2,3,5-triiodobenzoate) (see Davy et al. Polymer International 43:143-154 (1997)), 2,5-diiodo-8-quinolyl methacrylate (see Vazquez et al. Biomaterials 20:2047-2053 (1999)), and 4-iodophenyl methacrylate (see Kruft et al. J. Biomedical Materials Res. 28:1259-1266 (1994)). It is clear however that the resulting set cement will not only contain residual unreacted organoiodine monomer, but that exposure to physiological fluids will result in the release of organoiodine compounds with unclear physiological compatibility due to cleavage of ester bonds.

An alternative approach has been to incorporate in the bone cement particles of water-soluble X-ray contrast agents, e.g. iodixanol or iohexol (see Sabokbar et al. 46th Annual Meeting of the Orthopaedic Research Society, March 12-15, 2000, Orlando, Florida, page 0171). While avoiding the problem of the release of abrasive radioopaque particles from the bone cement, this approach however does not address the problem of impaired mechanical strength experienced with the conventional use of radioopaque particles of for example zirconia or barium sulphate.

Furthermore, neither of these approaches will produce cements in which the radioopaque material is substantially uniformly distributed in the set cement as, in order to reduce the amount of heat emitted on setting of the cement, it is conventional to use a two part cement using about 2 parts by weight of a preformed particulate polymer to about one part by weight of a liquid comprising monomer and a polymerization initiator.

We have now realized that these problems with radioopaque bone cements may be addressed by adopting one, two or three of the following strategies: firstly incorporating in the cement a non-polymerizable organoiodine compound which is soluble in the monomer from which the polymer matrix is produced; secondly by incorporating a polymerizable organoiodine monomer (or cross-linking agent) as part of the polymer matrix both in the part formed from the liquid monomer component and in the preformed polymer particles; and thirdly by the use of a polymerizable organoiodine monomer comprising an organoiodine group coupled via amide rather than ester bonds to at least one group copolymerizable with acrylic monomers. The resulting bone cement has been found to have a chemically homogeneous distribution within the particulate polymer portion and the polymer which is prepared in situ from the monomer

By polymerizable and non-polymerizable herein is meant that the compound in question can or respectively cannot participate in the polymerization reaction to become a covalently bound portion of the polymer matrix in the set bone cement.

Thus viewed from one aspect the invention provides a bone cement comprising in admixture a monomer-containing liquid portion and a particulate polymer portion, characterized in that at least one of said portions comprises a dissolved non-polymerizable organoiodine compound.

Viewed from a further aspect the invention comprises a bone cement kit comprising a monomer-containing liquid portion and separate therefrom a particulate polymer portion, wherein at least one of said portions comprises a dissolved non-polymerizable organoiodine compound, said kit optionally and preferably further comprising instructions for the preparation of a bone cement therewith.

Viewed from a still further aspect the invention provides a bone cement comprising in admixture a monomer-containing liquid portion and a particulate polymer portion, characterized in that said liquid portion comprises a polymerizable organoiodine compound and the polymeric structure of said particulate polymer comprises covalently bonded residues of a polymerizable organoiodine compound.

Viewed from another aspect the invention provides a bone cement kit comprising a monomer-containing liquid portion and separate therefrom a particulate polymer portion, wherein said liquid portion comprises a polymerizable organoiodine compound and the polymeric structure of said particulate polymer comprises covalently bonded residues of a polymerizable organoiodine compound, said kit optionally and preferably further comprising instructions for the preparation of a bone cement therewith.

Viewed from a further aspect the invention provides a bone cement comprising in admixture a monomer-containing liquid portion and a particulate polymer portion, characterized in that said liquid portion comprises a polymerizable organoiodine compound and/or the polymeric structure of said particulate polymer comprises covalently bonded residues of a polymerizable organoiodine compound, wherein said polymerizable organoiodine compound comprises an organoiodine moiety covalently bonded via an amide but not an ester bond to a polymerizable moiety.

Viewed from another aspect the invention provides a bone cement kit comprising a monomer-containing liquid portion and separate therefrom a particulate polymer portion, wherein said liquid portion comprises a polymerizable organoiodine compound and/or the polymeric structure of said particulate polymer comprises covalently bonded residues of a polymerizable organoiodine compound, wherein said polymerizable organoiodine compound comprises an organoiodine moiety covalently bonded via an amide but not an ester bond to a polymerizable moiety.

The cement or the cement components of the kit of the present invention preferably comprises an antibiotic compound.

More preferably the cement or the cement components of the kit of the present invention comprises an organoiodine compound and an antibiotic compound.

Especially preferably the cement or the cement components of the kit of the present invention comprises a chemically homogeneous distribution of all components within the final bone cement.

The antibiotic compound may be selected from gentamicin, colistin, erythromycin, clindamicin, penicillins, norfloxacin, chloramphenicol etc. Such antibiotics are included in several conventional bone cements and may be used in similar concentrations in the bone cement of the invention. Such an antibiotic may be in the form of a lipophilic ester, such as an acyl derivative e.g. an acetyl ester, whereby to allow its release from the cement over a prolonged period as a result of esterase activity in the physiological fluids contacting the cement after prosthesis implantation. A typical example of such an acyl derivative is clindamycin-2-polymerate ester. Alternatively, such antibiotics may be included functionalised to incorporate a polymerizable ethylenically unsaturated bond coupled via an ester group to the antibiotic moiety, whereby again to release the antibiotic moiety over a prolonged period as a result of esterase activity.

This use of antibiotic esters is novel and forms a further aspect of the invention.

The term "homogenous distribution" means that the chemical substances are present in both components ie. the particulate polymer phase and the polymer which is prepared in situ from the monomer during use, more preferably present in both components in concentrations which differ by less than 50%, more preferably by less than 20%, even more preferably by less than 10%.

Most preferred standard chemical analytical techniques such as GC analysis, HPLC analysis and CE analysis are not able to identify qualitative or quantitative differences in the distribution of the chemicals ie. organoiodine compounds and antibiotics within the final bone cement.

The organoiodine compound(s) used in the present invention are preferably iodobenzene compounds (i.e. having iodine as a ring substituent on an aromatic C₆ ring), especially diiodo or more preferably triiodo benzene compounds, in particular diiodo and triiodo benzene compounds having the iodines at non-adjacent ring positions. The iodobenzene ring is preferably also substituted by one, two or three substituent groups comprising non-polymerizable lipophilic groups and/or polymerizable groups, preferably acrylate or methacrylate groups, and/or polymerizable or non-polymerizable groups coupled to a further iodobenzene ring (preferably a triiodobenzene ring). The non-polymerizable lipophilic groups are preferably acrylamino or alkylaminocarbonyl groups, or more preferably acyloxyalkylcarbonylamino, acyloxyalkylaminocarbonyl, alkoxyalkylaminocarbonyl or alkoxyalkylcarbonylamino groups, i.e. groups which on hydrolysis (for example due to esterases encountered in the body) will serve to solubilize the iodobenzene moiety, e.g. groups of formula

(R¹COO) ₙXCONR²-

or

(R¹COO) ₙXNR²CO-

or

((R¹COO) ₙX) ₂NCO-

or

(R³O) ₙXCONR²-

or

(R³O) ₙXNR²CO-

or

((R³O) ₙX) ₂NCO-

(where n is 1, 2, 3 or 4, preferably 2 or 3;
R¹ is a C₁₋₆ alkyl group, especially a methyl group;
X is a C₁₋₆ alkylene group, especially an ethylene and
more especially a propylene group;
R² is hydrogen or a C₁₋₆ alkyl group, especially hydrogen or a methyl group, and R³ is a C₁₋₆ alkyl group,
especially a methyl group, or two groups R³ together form a C₁₋₆ alkanediyl group, e.g. a propan-2,2-diyl group).

Particularly preferably, the organoiodine compound is a compound of formula I or II 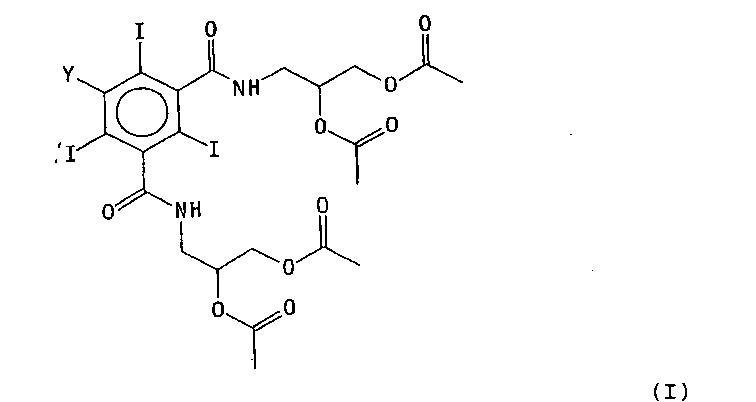 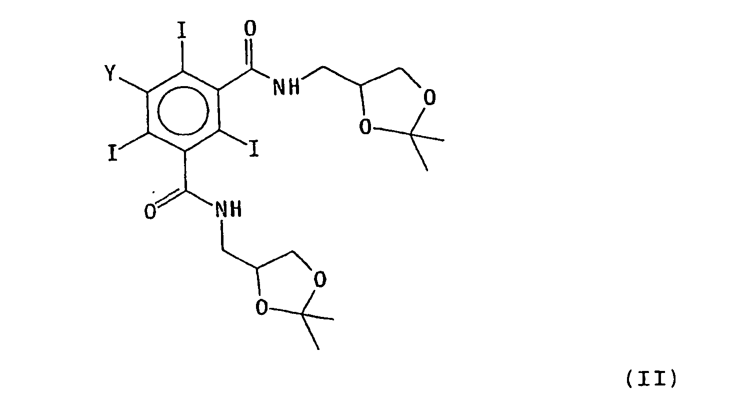 where Y is a group -NHCOCH₃,
-CONH-CH₂CH (OCOCH₃) CH₂ (OCOCH₃),
-NHCOCH₂CH (OCOCH₃) CH₂ (OCOCH₃), 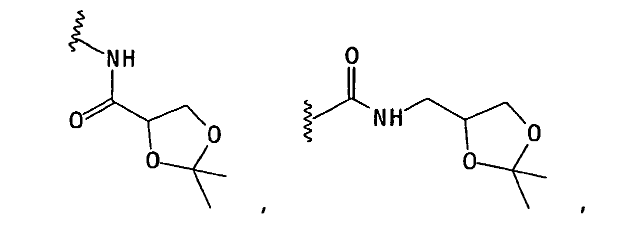 -NH-CO-C (CH₃) =CH₂, -NH-CO-NH-D-NH-CO-C (CH₃) =CH₂,
-NH-CH₂-CO-O-D-CO (CH₃) =CH₂,
-NH-CH₂-CO-NH-D-NH₂-CO-C (CH₃) =CH₂,
-CO-NH-D-NH-CO-C(CH₃)=CH₂, or -CO-O-D-O-CO-C(CH₃)=C_{H2},
i.e. a compound which is non-polymerizable but soluble in acrylic monomers or a compound which is polymerizable with acrylic monomers respectively.

As mentioned above, the organoiodine compound may be a bis-iodobenzene compound. Examples of such compounds include those of formula III 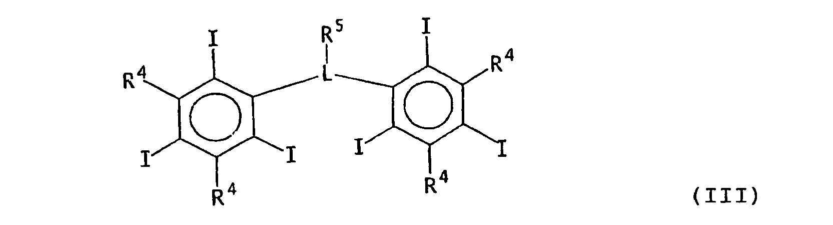 where R⁴ is a lipophilic non-polymerisable group (e.g. a group (R¹COO)ₙX CONR²-, etc as defined above, in particular a group -CONHCH₂CH(OCOCH₃) CH₂ (OCOCH₃) or 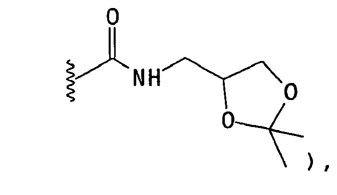 L is a linker group providing a 3 to 5, preferably 3, atom bridge between the phenyl rings, preferably a nitrogen attached bridge (e.g. a -N-C-N-bridge), and R⁵ is a polymerizable group, e.g. a (meth)acrylate or (meth)acrylamide (e.g. CH₂=C(CH₃)COO or CH₂=C(CH₃)CONH) group optionally attached via a linker, e.g. a C₁₋₆ alkanediyl group.

Alternative preferred non-polymerizable and polymerizable organoiodine compounds include analogs of known non-ionic, monomeric or dimeric organoiodine X-ray contrast agents in which solubilizing hydroxyl groups are acylated (e.g. acetylated) or formed into 2,4-dioxacyclopentan-1-yl groups and/or, where the compound is to be polymerizable, in which a carbonyl- or nitrogen-attached ring substituent is replaced by a (meth)acrylamide group or a (meth)acrylamidoalkylamino carbonyl group).

Examples of conventional non-ionic X-ray contrast agents which may be modified in this way include: iohexol, iopentol, iodixanol, iobitridol, iomeprol, iopamidol, iopromide, iotrolan, ioversol and ioxilan. The use of the analogs of the contrast agents with regulatory approval (e.g. in the US, Japan, Germany, Britain, France, Sweden or Italy) is preferred. The use of the analogs of the monomeric contrast agents is particularly preferred. Such analogs may be prepared by acylation (e.g. acetylation) of the contrast agent itself or of an aminobenzene precursor and, where the analog is to be polymerized, by subsequent reaction of an optionally activated alkeneoic acid (e.g. an alkeneoic acid chloride (for example methacrylic acid chloride)) with an aminobenzene precursor. The preparation of such contrast agents and their aminobenzene precursors is widely described in the patent and scientific literature.

The use of such analogs is especially advantageous as any organoiodine compound released from the bone cement, e.g. due to esterase activity of biological fluids, will be in the form of a physiologically tolerable compound or a compound with biodistribution, bioelimination and biotolerability closely similar to the known and approved contrast agents. Before such exposure to esterase activity, the lipophilic acyl groups will moreover serve to reduce any leaching of the organoiodine compound from the bone cement.

The organoiodine compound, i.e. a monomer and/or a monomer soluble compound, may constitute up to 100% wt of the liquid or particulate portion if a monomer but generally will constitute 2 to 75% wt, more preferably 5 to 50% wt, especially 10 to 25% wt of the portion(s) it is present in. Especially preferably it is present in both portions, and particularly at a weight percentage in the liquid portion which is within 5% wt, especially within 2% wt, of the weight percentage in the particulate portion.

If desired, some or all of the organoiodine compound may take the form of a cross-linking agent carrying at least two and optionally up to 10 or more polymerizable groups (e.g. (meth)acrylic groups). Generally however such cross-linking agents will constitute only a minor proportion, e.g. up to 20% (on a molar iodine basis) of the total organoiodine compound used, more preferably up to 10%, especially up to 5%. Such cross-linking agents may conveniently be prepared by reacting conventional X-ray contrast agents of the types mentioned above or their aminobenzene precursors (or partly acylated versions of either thereof) with an optionally activated alkeneoic acid (e.g. methacrylic acid chloride).

Less preferably, the organoiodine compound may be an iodobenzene free from non-polymerizable lipophilic substituents (other than iodine of course), e.g. a simple iodobenzene (such as 1,4-diiodobenzene) or a simple iodoaminobenzene conjugate with (meth)acrylic acid (e.g. methacrylamido-2,4,6-triiodobenzene). Besides the organoiodine compound (where present), the liquid portion of the cement composition will comprise at least one polymerizable monomer, generally a monomer containing an ethylenically unsaturated bond, optionally a polymerization initiator, and optionally a cross-linking agent. The polymerization initiator and cross-linking agent may if desired be added to the liquid monomer or the liquid/particle mixture during preparation of the cement for use. Examples of suitable monomers include for example acrylic acid, methyl acrylate, ethyl acrylate, methacrylic acid, methyl methacrylate, butyl methacrylate and styrene. Especially preferably the monomer is methyl methacrylate, optionally together with butyl methacrylate, e.g. in a 4:1 to 10:1 ratio by weight, especially a 4.5:1 to 7:1 ratio.

Certain of the organoiodine compounds used according to the invention are themselves novel and form a further aspect of the invention. Thus viewed from this aspect the invention provides an organoiodine compound of formula IV 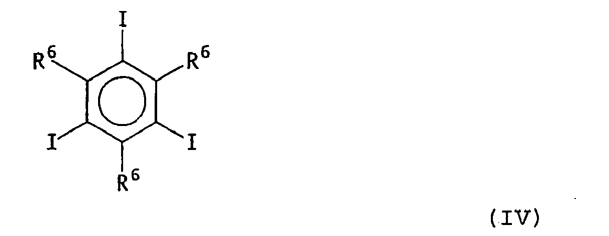 wherein each R⁶ group which may be the same or different, comprises an acyloxyalkylcarbonylamino, N-(acyloxyalkyl carbonyl)acyloxyalkylamino, N-acyloxyalkylcarbonyl-N-alkyl-amino, acyloxyalkylaminocarbonyl,
bis(acyloxyalkyl)aminocarbonyl, N-acyloxyalkyl-N-alkylaminocarbonyl, alkoxyalkylaminocarbonyl, N-alkylalkoxyalkylaminocarbonyl, bis(alkoxyalkyl)aminocarbonyl, alkoxyalkylcarbonylamino, N-alkylalkoxyalkylcarbonylamino or N-alkoxyalkylcarbonylalkoxyalkylamino group or a triiodophenyl group attached via a 1 to 10 atom bridge (preferably composed of bridging atoms selected from O, N and C) optionally substituted by an acyloxyalkyl, acyloxyalkylcarbonyl, acyloxyalkylamino, acyloxyalkylcarbonylamino, acyloxyalkylaminocarbonyl, alkoxyalkyl, alkoxyalkylcarbonyl, alkoxyalkylamino, alkoxyalkylcarbonylamino, or alkoxyalkylaminocarbonyl group or by a polymerizable group, e.g. a (meth)acrylate or (meth)acrylamide group, or one or two R⁶ groups is/are a polymerizable group, e.g. a (meth)acrylate or (meth)acrylamide group, optionally attached via a 1 to 10 atom bridge, e.g. an alkylaminocarbonyl or alkylcarbonylamino bridge; or where one R⁶ group is a polymerizable group, one or both of the remaining R⁶ groups may be an alkylamino, bisalkylamino, alkylcarbonylamino, N-alkyl-alkylcarbonylamino, alkylaminocarbonyl or bis-alkyl-aminocarbonyl group, (e.g. an acetylamino group). In such compounds, any alkyl or alkylene moiety preferably contains 1 to 6 carbon atoms, especially 2 to 4 carbon atoms and any bridge optionally comprises oxygen and/or nitrogen atoms, especially one or two nitrogen atoms. Moreover, two alkoxy groups in such compounds, especially groups attached to neighbouring carbon atoms, may be fused to form a cyclic bis-ether, preferably containing two ring oxygens and three ring carbons, e.g. as a 2,4-dioxa-3,3-dimethyl-cyclopentan-1-yl group. In general, it is preferred that two R⁶ groups are carbonyl-attached and that one is nitrogen-attached to the iodobenzene ring.

The compounds according to the invention may be prepared from triiodophenyl carboxylic acids and amines, e.g. conventional intermediates in X-ray contrast agent production, by protection of hydroxy groups and reaction of the carboxyl group (optionally after activation) or amino group with a polymerizable amine or carboxylic acid or activated carboxylic acid optionally subsequent to reaction with a spacer molecule, e.g. a diol or diamine.

The polymerization initiator, where present, is preferably a physiologically tolerable initiator of polymerization of ethyleneically unsaturated monomers, e.g. N,N-dimethyl-p-toluidine, N,N-dimethylaminobenzyl alcohol (DMOH) or N,N-dimethylaminobenzyl oleate (DMAO). This initiator is conveniently included as 0.1 to 5% wt of the liquid monomer composition, preferably 1 to 3% wt.

If a cross-linking agent (e.g. an organoiodine compound containing 2 or more polymerizable ethylenic bonds, divinylbenzene, ethyleneglycoldimethylacrylate, etc) is present in the liquid monomer composition, this is preferably as up to 2% wt of the composition, especially 0.1 to 1% wt of the composition.

As with conventional liquid monomer portions of bone cements, the portion may contain further components, e.g. hydroquinone, growth hormone, BMP (Bone Morphogenic Protein), vitamins (e.g. vitamin D and vitamin E), etc.

The liquid monomer portion conveniently comprises 25 to 45% wt of the cement, especially preferably 30 to 35% wt.

The second component of the bone cement of the invention is a particulate polymer. This may be prepared by any conventional polymerization process, e.g. emulsion, suspension, liquid, slurry or gas phase polymerization, but is preferably prepared by emulsion polymerization or by the swelling and polymerization process pioneered by Prof. Ugelstad of NTNU, Norway (see the patent and scientific publications by Ugelstad and by Sintef/Sinvent).

In an especially preferred embodiment of the invention the polymer particles are prepared by emulsion polymerization. Preferably said emulsion is an aqueous emulsion i.e. oil-in-water emulsion. The aqueous phase preferably contains an emulsifier or an agent which improves the dispersion and stabilizes the emulsion of the monomer during polymerization. Such agents are well known in the prior art e.g. a polymer such as polyvinylalcohol or a low molecular weight emulsifier such as polysorbate 80.

A further aspect of the present invention is the method used to emulsify the monomer in the homogeneous phase during emulsion polymerization. The mixture can be stirred, shaken or more effectively treated with a homogenizer such as a stator-rotor system.

The polymerization temperature can vary over a large range. Preferably the polymerization temperature is in the range of 50-100°C; more preferably 60-90°C; especially preferably 70-80°C.

The polymerization reaction time can also vary over a large range. Preferably the reaction time is in the range from 4 hours to 5 days, more preferably 6 hours to 2 days.

All these parameters affect the physical and chemical properties of the polymer particles formed i.e. molecular weight, particle size, monomer content etc.

In a further aspect of the present invention regarding the preparation of polymer particles with contrast agents and/or antibiotic agents salts are added to the aqueous phase to increase the content of the contrast agents and/or antibiotic agents within the polymer particles.

In an even further aspect of the present invention regarding the preparation of polymer particles with contrast agents and/or antibiotic agents the pH is adjusted so as to increase the content of the contrast agents and/or antibiotic agents within the polymer particles. The pH is adjusted by the addition of acids or bases or by the use of buffer.

The monomers and, where used, the organoiodine components and cross-linking agents, used in the preparation of the particulate polymer portion of the bone cement may be any of the materials described above in connection with the liquid monomer portion.

Particularly preferably, the monomer and, where present, the organoiodine compound used in the preparation of the particulate polymer portion are the same as are described above for use in the liquid monomer composition.

The polymerization initiator used in the preparation of the particulate polymer portion may be any compound suitable for polymerization initiation. Examples of suitable initiators include benzyl peroxide

(BPO), 2,2'-azo-bis-isobutyronitrile (AIBN) and tert butyl peroxybenzoate. Preferably however benzoyl peroxide is used.

The relative concentrations of organoiodine compound and monomer used in the preparation of the particulate polymer portion are preferably as described above for the liquid monomer portion; especially preferably they are the same as in the liquid monomer portion.

The particulate polymer portion preferably has a mode particle size (i.e. maximum particle dimension in any direction)' of 1 to 200 µm, particularly 2 to 100 µm, especially 10 to 70 µm; the particulate may be substantially monodisperse, e.g. with a coefficient of variation of less than 10%, especially less than 5%.

In a preferred embodiment of the invention if the average particle size is large the particles are polydisperse.

The particulate portion may, but generally will not, contain further components such as the antibiotics (in particular clindamycin, gentamicin, colistin, erythromycin, penicillins, norfloxacin and chloramphenicol), growth hormones, BMP (Bone Morphogenic Protein), vitamins, etc mentioned above.

As with conventional bone cements, the cement composition of the invention should be mixed in a way that minimizes incorporation of gas bubbles into the set cement. Typically this will involve mixing the liquid and particulate portions under reduced pressure. However in a preferred embodiment mixing is effected in a helium atmosphere, especially preferably using portions that have been degassed and then helium flushed and if appropriate packed in a helium atmosphere. This use of helium in bone cement preparation is novel and forms a further aspect of the present invention. Viewed from this aspect the invention provides a method of producing a bone cement comprising admixing a liquid monomer portion and a particulate polymer portion, characterized in that admixture of said portions is effected under helium.

In a further aspect the invention provides a method of affixing a joint prosthesis comprising inserting said prosthesis and a bone cement into a bone cavity, characterized in that said cement is a cement according to the invention.

The bone cements according to the invention are especially beneficial as compared to conventional zirconia-containing bone cements due to their X-ray opacity at the enhanced irradiation intensities necessary for investigation of joint prosthesis.

In a further aspect of the invention, in place of the organoiodine compounds, directly analogous organobromine compounds may be used. The resulting cements are especially useful as dental cements and as bone cements for thin extremities where lower X-ray voltages may be used.

Viewed from a still further aspect the invention provides bone cement characterized in that the mechanical properties regarding the ultimate tensile strength and ultimate strain are greater than 10% higher than these of Palacos^{®} bone.

Bone cement compositions additionally comprising antibiotic compounds themselves form a further aspect of the invention.

Bone cement compositions comprising a chemically homogeneous distribution of all components within the final cement also form a further aspect of the invention.

These bone cement compositions can be sterilized by ethylene oxide or gamma radiation.

The invention will now be described further with reference to the following non-limiting Examples. Parts and percentages are by weight unless otherwise indicated.

### Example 1

### Synthesis of iohexol-O-hexaacetate

Iohexol (24.63 g, 30.00 mmol) was suspended in a solution of acetic anhydride (100 ml) and pyridine (20 ml). The mixture was stirred for 24 hours at ambient temperature and then evaporated *in vacuo,* diluted with CH₂Cl₂ (200 ml) and washed with 0.1 M HCl (3 x 50 ml). The organic layer was dried with anhydrous MgSO₄, filtered and evaporated *in vacuo* producing a white crystalline solid (28.23 g, 87.67%).

| | |
|---|---|
| ¹H NMR | 6.88-6.77 (m, 1 H), 5.31-5.20 (m, 3 H), 4.39- 3.56 (m, 12 H), 2.06-2.00 (m, 18 H), 1.86 (d, 3 H) |
| MS(ES) | 1095.9 (M+Na⁺, 100) |

### Example 2

### Synthesis of iodixanol-O-nonaacetate

Iodixanol (4.65 g, 3.00 mmol) was suspended in a solution of acetic anhydride (20 ml) and pyridine (4 ml). The reaction mixture was stirred for 24 hours and then evaporated *in vacuo,* diluted with CH₂Cl₂ (40 ml) and washed with 0.1 M HCl (3 x 25 ml). The organic layer was dried with anhydrous MgSO₄, filtered and evaporated *in vacuo* producing a white crystalline solid (5.27 g, 91.1%).

| | |
|---|---|
| ¹H NMR | 6.66-6.44 (m, 4 H), 5.19 (br s, 5 H), 4.37- 3.50 (m, 20 H), 2.04 (s, 18 H), 1.86-1.72 (m, 9 H), 1.21 (s, 6 H) |
| MS(ES) | 1950.8 (M+Na⁺, 100), 439.2 (18) |

### Example 3

### Synthesis of 5-amino-2,4,6-triiodo-N,N'-bis(2,3-dihydroxyoropyl)-isophthalamide bis-acetonoid

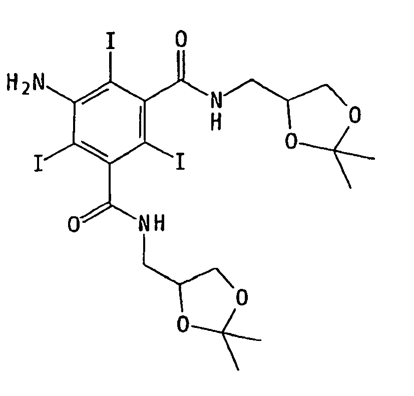

5-Amino-2,4,6-triiodo-N,N'-bis(2,3-dihydroxypropyl)-isophthalamide (10.00 g, 14.18 mmol) (an intermediate in the preparation of iohexol) and toluidine sulphonic acid (TsOH) (0.10 g, 0.52 mmol) were added to 2,2-dimethoxypropane (30 ml). The suspension mixture was stirred at ambient temperature for 48 hours and the ethanol formed was distilled off. The reaction mixture was evaporated in vacuo to yield a white solid (R_{F}=0.45 ethyl acetate).

¹H-NMR (DMSO): 8.70-8.64 (m, 2H), 4.24-4.20 (m, 2H), 4.05 (t, 2H), 3.83-3.77 (m, 2H), 3.43-3.16 (m, 4H), 1.34 (s, 6H), 1.26 (s, 6H).

### Example 4

### Synthesis of 5-(propen-2-yl-carbonylamino)-2,4,6-triiodo-N,N'-bis(2 3-dihydroxypropyl)-isophthalamide bis-acetonoid

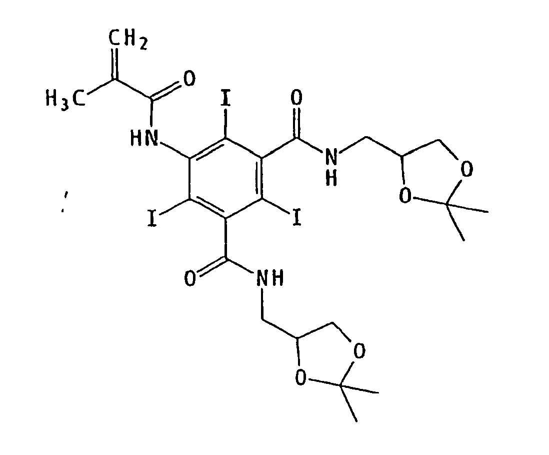

To the acetonoid product of Example 3 (1.57 g, 2.00 mmol) in ethylacetate (25 ml) and pyridine (1 ml) was added methacryloyl chloride (0.21 g, 2.00 mmol). The mixture was stirred at ambient temperature for 12 hours. The reaction mixture was added to ethylacetate (35 ml) and washed with water (3x15 ml). The organic layer was dried with MgSO₄, filtered and evaporated in vacuo to yield a white crystalline solid.

¹H-NMR (DMSO): 8.70-8.64 (m, 2H), 6.23 (s, 1H), 6.07 (s, 1H), 4.24-4.20 (m, 2H), 4.05 (t, 2H), 3.83-3.77 (m, 2H), 3.43-3.16 (m, 4H), 1.87 (s, 3H), 1.34 (s,6H), 1.26 (s, 6H).

### Example 5

### Synthesis of the chloroacetate ester of 2-hydroxy-methylmethacrylate

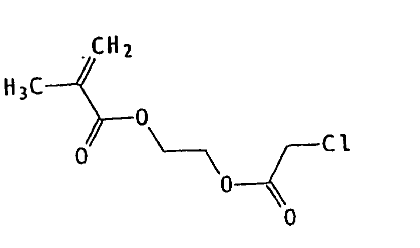

Chloroacetoylchloride (2.25 g, 20.00 mmol) in CH₂Cl₂ (10 ml) was added dropwise to a solution of hydroxyethyl methylmethacrylate (HEMA) (2.60 g, 20.00 mmol) and N (C₂H₅)₃ (2.02 g, 20.00 mmol) in CH₂Cl₂ (20 ml) at 0°C and stirred at ambient temperature overnight. The reaction mixture was added to CH₂Cl₂ (20 ml) and the mixture was washed with saturated NaHCO₃ (2x25 ml) and 1M HCl (25 ml), dried with MgSO₄, filtered and evaporated in vacuo to yield a yellow oil.

¹H-NMR (CDCl₃): 6.05 (2, 1H), 5.54 (s, 1H), 4.39-4.29 (m, 4H), 4.02 (s, 2H), 1.87 (s, 3H)

¹³C-NMR (CDCl₃): 167.04, 166.87, 135.63, 126.10, 63.59, 61.85, 40.54, 18.05.

### Example 6

### Synthesis of 5-(propen-2-ylcarbonyloxyethyloxycarbonylmethylamino)-2,4,6-triiodo-N,N'-bis(2,3-dihydroxypropyl)-isophthalamide bis-acetonoid

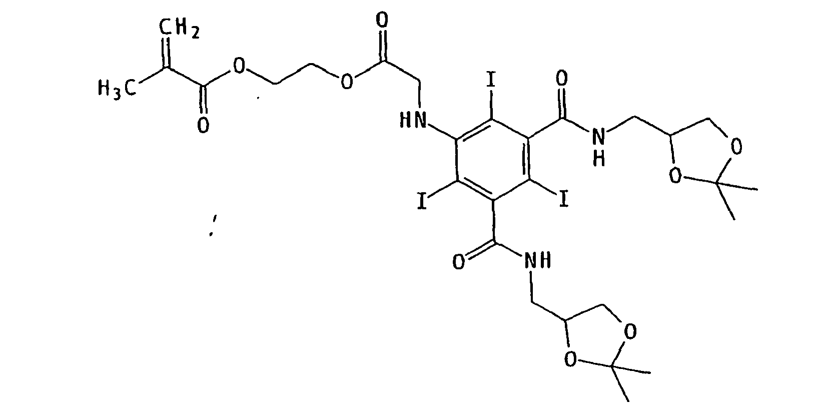

The compound of Example 5 (0.41 g, 2.00 mmol) in tetrahydrofuran (THF) was added dropwise to a solution of the acetonoid of Example 3 (1.57 g, 2.00 mmol) and N(C₂H₅)₃ (0.20 g, 2.00 mmol) in THF (20 ml). The mixture was stirred at ambient temperature for 24 hours. The reaction mixture was then evaporated down in vacuo, ethylacetate (30 ml) and H₂O (20 ml) were added and the mixture was transferred to a separation funnel. The aqueous layer was repeatedly extracted with ethylacetate (2x25 ml), dried with MgSO₄, filtered and evaporated in vacuo to yield a white crystalline powder.

¹H-NMR (CDCl₃): 7.24 (d, 2H), 6.06 (s, 1H), 5.54 (s, 1H), 4.39-4.32 (m, 6H), 4.06-4.01 (m, 4H), 3.78-3.43 (m, 6H), 1.79 (s, 3H), 1.36 (s, 6H), 1.23 (s, 6H).

### Example 7

### Synthesis of triiodophenyl dimer

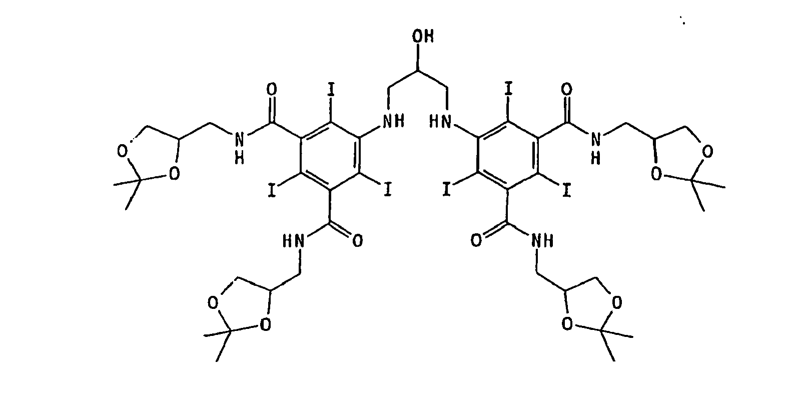

The acetonoid of Example 3 (3.14 g, 4.00 mmol) and K₂CO₃ (2.21 g, 16.00 mmol) in acetonitrile (40 ml) were added to 1,3-dibromo-2-propanol (0.44 g, 2.00 mmol) and the mixture was heated at 100°C for 24 hours, cooled to ambient temperature and filtered. The filtrate was evaporated in vacuo to yield a white crystalline solid.

¹H-NMR (DMSO): 8.71-8.57 (m, 4H), 5.48 (m, 1H), 4.30-4.23 (m, 4H), 4.10 (t, 4H), 3.87-3.79 (m, 4H), 3.40-3.23 (m, 12H), 1.38 (s, 12H), 1.29 (s, 12H)

### Example 8

### Synthesis of triiodophenyl dimer derivative

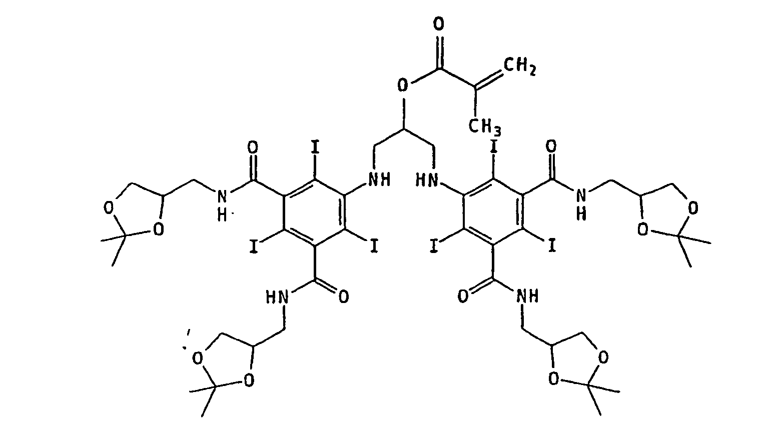

To the triiodophenyl dimer of Example 7 (1.62 g, 1.00 mmol) and N(C₂H₅)₃ (0.12 g, 1.2 mmol) in acetonitrile (15 ml) was added methacryloyl chloride (0.19 g, 1.2 mmol) in acetonitrile (5 ml). The mixture was stirred at ambient temperature for 12 hours. The mixture was then evaporated down in vacuo, ethylacetate (30 ml) was added and the resultant mixture was washed with H₂O (3x20 ml), dried with MgSO₄, filtered and evaporated in vacuo to yield a yellow crystalline solid.

¹H-NMR (DMSO): 8.71-8.45 (m, 4H), 6.23 (s, 1H), 6.01 (s, 1H), 5.45 (m, 1H), 4.25-4.21 (m, 4H), 4.05 (t, 4H), 3.82-3.77 (m, 4H), 3.56-3.18 (m, 12H), 1.93 (s, 3H), 1.34 (s, 12H), 1.25 (s, 12H)

### Example 9

### Synthesis of Polymerizable amide derivative with spacer

Diatrizoic acid is reacted with oxalyl chloride (1 equivalent) in dimethylformamide (DMF) to form the corresponding acid chloride. The acid chloride is then further reacted with ethylenediamine to form the corresponding monoamide. The monoamide is then reacted with methacrylic acid chloride and the resulting amide acryl derivative is isolated by chromatography.

### Example 10

### Polymerization of MMA to form PMMA particles containing 10% iohexol-hexaacetate

A solution of 0.5% polyvinylalcohol (PVA) (mw 15000) in water (300 ml) was heated to 80°C and the solution was "bubbled" with argon. A solution of methylmethacrylate (44.75 g), iohexol-hexaacetate (5.0 g) (from Example 1) and benzoylperoxide (0.25 g) was added dropwise to the solution over a period of 30-60 minutes. The polymerization mixture was refluxed for 2 days, cooled to ambient temperature and filtered. The solid was dried overnight at 60°C (44.5 g).

### Example 11

### Polymerization of MMA to form PMMA particles containing 6.67% iohexol-hexaacetate

A solution of 0.5% polyvinylalcohol (PVA) (mw 15000) in water (300 ml) was heated to 80°C and the solution "bubbled" with argon. A solution of methylmethacrylate (46.41 g), iohexol-hexaacetate (3.33 g) (from Example 1) and benzoylperoxide (0.25 g) was added dropwise to the solution over a period of 30-60 minutes. The polymerization mixture was refluxed for 2 days, cooled to ambient temperature and filtered. The solid was dried overnight at 60°C (43.9 g).

### Example 12

### Polymerization of MMA to form PMMA particles

A solution of 0.5% polyvinylalcohol (PVA) (mw 15000) in water (300 ml) was heated to 80°C and the solution "bubbled" with argon. A solution of methylmethacrylate

(49.75 g) and benzoylperoxide (0.25 g) was added dropwise to the solution over a period of 30-60 minutes.

The polymerization mixture was reflexed for 2 days, cooled to ambient temperature and filtered. The solid was dried overnight at 60°C (45.6 g).

### Example 13

### Polymerization of MMA to form PMMA particles containing 10% iodixanol-nonaacetate

A solution of 0.5% polyvinylalcohol (PVA) (mw 15000) in water (100 ml) was heated to 80°C and bubbled with argon. Iodixanol-nonaacetate (1 g) (from Example 2) was mixed with methylmethacrylate (9 g) under ultrasonic radiation, benzoylperoxide (50 mg) was added and the resultant mixture was added dropwise to the solution over a period of 30-60 minutes. This polymerization mixture was refluxed for 2 days, cooled to ambient temperature and filtered. The solid product was dried overnight at 60°C (7.58 g).

### Example 14

### Synthesis of PMMA particles containing an iohexol-derivative

A solution of 0.5% polyvinylalcohol (PVA) (mw 15000) in water (100 ml) was heated to 80°C and bubbled with argon. The polymerizable acetonoid of Example 6 (0.477 g, 0.5 mmol) was mixed with methylmethacrylate (9.47 g, 94.6 mmol) under ultrasonic radiation, benzoylperoxide (50 mg) was added and the mixture was added dropwise to the solution over a period of 30-60 minutes. The polymerization mixture was refluxed for 2 days, cooled to ambient temperature and centrifuged at 4000 rpm for 10 minutes. The supernatant was removed, and the centrifugate was washed with water. The product was dried at 50°C to yield a white solid.

### Example 15

### Synthesis of PMMA particles containing an iohexol dimer

A solution of 0.5% polyvinylalcohol (PVA) (mw 15000) in water (100 ml) was heated to 80°C and bubbled with argon. The polymerizable dimer of Example 8 (0.87 g, 0.5 mmol) was mixed with methylmethacrylate (9.07 g, 90.6 mmol) under ultrasonic radiation, benzoylperoxide (50 mg) was added and the mixture was added dropwise to the solution over a period of 30-60 minutes. The polymerization mixture was refluxed for 2 days, cooled to ambient temperature and centrifuged at 4000 rpm for 10 minutes. The supernatant was removed, and the centrifugate was washed with water. The product was dried at 50°C to yield a white solid.

### Example 16

### Polymerization of MMA to form PMMA particles containing 5% ethyl-4-iodobenzoate

A solution of 0.5% polyvinylalcohol (PVA) (mw 15000) in water (24 ml) was heated to 70°C and bubbled with argon. A solution of methylmethacrylate (3.78 g), ethyl-4-iodobenzoate (200 mg) and benzoylperoxide was added dropwise to the PVA solution over a period of 30-60 minutes. The polymerization mixture was refluxed for 3 days, cooled to ambient temperature and centrifuged at 4000 rpm for 10 minutes. The supernatant was removed, and the centrifugate was washed with water. The product was dried in vacuo at 50°C to a white solid.

### Example 17

### Polymerization of MMA to form PMMA particles containing 5% 1,2-diiodotetrafluorobenzene

A solution of 0.5% polyvinylalcohol (PVA) (mw 15000) in water (24 ml) was heated to 70°C and bubbled with argon. A solution of methylmethacrylate (3.78 g), 1,2-diiodotetrafluorobenzene (200 mg) and benzoylperoxide (20 mg) was added dropwise to the PVA solution over a period of 30-60 minutes. The polymerization mixture was refluxed for 3 days, cooled to ambient temperature and centrifuged at 4000 rpm for 10 minutes. The supernatant was removed, and the centrifugate was washed with water. The product was dried in vacuo at 50°C to a white solid.

### Example 18

### Polymerization of MMA to form PMMA particles containing 10% iohexol-hexaacetate

A solution of 0.5% polyvinylalcohol (PVA) (mw 15000) in water (300 ml) was heated to 70°C and bubbled with argon. A solution of methylmethacrylate (11.88 g), iohexol-O-hexaacetate (Example 1) (1.25 g) and benzoylperoxide (63 mg) was added dropwise to the PVA solution over a period of 30-60 minutes. The reaction mixture was stirred using a sonicator (ULTRA-TURRAX T 25 basic) at 11000 rpm. The polymerization mixture was refluxed for 3 days, cooled to ambient temperature and centrifuged at 4000 rpm for 10 minutes. The supernatant was removed, and the centrifugate was washed with water. The product was dried in vacuo at 50°C to a white solid. The particle size of the product was much lower than that of Example 10.

### Example 19

### Synthesis of PMMA particles containing a polymerizable iohexol-derivative

A solution of 0.5% polyvinylalcohol (PVA) (mw 15000) in water (100 ml) is heated to 80°C and bubbled with argon. The polymerizable iohexol derivative of Example 4 (0.477 g) is mixed with methylmethacrylate (9.47 g) under ultrasonic radiation, benzoylperoxide (50 mg) is added and the mixture is added dropwise to the PVA solution over a period of 30-60 minutes. The polymerization mixture is refluxed for 2 days, cooled to ambient temperature and filtered.

### Example 2 0

### Synthesis of PMMA particles containing a polymerizable iohexol-dimer

A solution of 0.5% polyvinylalcohol (PVA) (mw 15000) in water (100 ml) is heated to 80°C and bubbled with argon. The polymerizable dimer of Example 8 (0.87 g) is mixed with methylmethacrylate (9.07 g) under ultrasonic radiation, benzoylperoxide (50 mg) is added and the mixture is added dropwise to the PVA solution over a period of 30-60 minutes. The polymerization mixture is refluxed for 2 days, cooled to ambient temperature and filtered.

### Example 21

### Polymerization of MMA to form PMMA particles containing iohexol-O-hexaacetate and norfloxacin

A solution of 1.0 % polyvinylalcohol (PVA) (mw 15000) in water (80 ml) was heated to 80 °C and the solution "bubbled" with argon. A solution of methyl methacrylate (9.0 g), iohexol-O-hexaacetate (Example 1, 1.0 g), norfloxacin (500 mg) and benzoylperoxide (200 mg) was added dropwise to the solution of a period of 30-60 minutes. The mixture was vigorously stirred for 24 hours at 80 °C. The particulate material was isolated by centrifugation (4000 xg for 15 minutes), resuspended in water and isolated by centrifugation. The powder was dried under vacuum at 50 °C for 12 hours. Yield: 7.1 g. HPLC analysis: iohexol-O-hexaacetate 4.3 %

### Example 22

### Polymerization of MMA to form PMMA particles containing iohexol-O-hexaacetate and amoxicillin

A solution of 1.0 % polyvinylalcohol (PVA) (mw 15000) in water (80 ml) was heated to 80 °C and the solution "bubbled" with argon. A solution of methyl methacrylate (Example 1, 9.0 g), iohexol-O-hexaacetate (1.0 g), amoxicillin trihydrate (500 mg) and benzoylperoxide (200 mg) was added dropwise to the solution of a period of 30-60 minutes. The mixture was vigorously stirred for 24 hours at 80 °C. The particulate material was isolated by centrifugation (4000 xg for 15 minutes), resuspended in water and isolated by centrifugation. The powder was dried under vacuum at 50 °C for 12 hours. Yield: 13.8 g. HPLC analysis: iohexol-O-hexaacetate 2.4 %, amoxicillin 3.2 %

### Example 23

### Polymerization of MMA to form PMMA particles containing iohexol-O-hexaacetate and chloramphenicol

A solution of 1.0 % polyvinylalcohol (PVA) (mw 15000) in water (80 ml) was heated to 80 °C and the solution "bubbled" with argon. A solution of methyl methacrylate (9.0 g), iohexol-O-hexaacetate (Example 1, 1.0 g), chloramphenicol (50 mg) and benzoylperoxide (200 mg) was added dropwise to the solution of a period of 30-60 minutes. The mixture was stirred vigorously for 24 hours at 80 °C. The particulate material was isolated by centrifugation (4000 xg for 15 minutes), resuspended in water and isolated by centrifugation. The powder was dried under vacuum at 50 °C for 12 hours. Yield: 6.0 g. HPLC analysis: iohexol-O-hexaacetate 4.5 %, chloramphenicol 0.28 %

### Example 24

### Polymerization of MMA to form PMMA particles containing iohexol-O-hexaacetate and gentamycin sulfate

A solution of 2.0 % polyvinylalcohol (PVA) (mw 15000) in water (80 ml) was heated to 80 °C and the solution "bubbled" with argon. A solution of methyl methacrylate (9.0 g), iohexol-O-hexaacetate (Example 1, 1.0 g), gentamycin sulfate (500 mg) and benzoylperoxide (200 mg) was added dropwise to the solution of a period of 30-60 minutes. The mixture was stirred vigorously for 24 hours at 80 °C. The particulate material was isolated by centrifugation (4000 xg for 15 minutes), resuspended in water and isolated by centrifugation. The powder was dried under vacuum at 50 °C for 12 hours. Yield: 5.45 g. HPLC analysis: iohexol-O-hexaacetate 4.6 %. Gentamycin sulfate was qualitativly detected by HPLC

### Example 25

### Polymerization of MMA to form PMMA particles containing iohexol-O-hexaacetate and gentamycin sulfate

A solution of 2.0 % polyvinylalcohol (PVA) (mw 15000) in water (80 ml) containing sodium chloride (20 g) was heated to 80 °C and the solution "bubbled" with argon. A solution of methyl methacrylate (9.0 g), iohexol-O-hexaacetate (Example 1, 1.0 g), gentamycin sulfate (500 mg) and benzoylperoxide (200 mg) was added dropwise to the solution of a period of 30-60 minutes. The mixture was stirred for 24 hours at 80 °C. The particulate material was isolated by centrifugation (4000 xg for 15 minutes), resuspended in water and isolated by centrifugation. The powder was dried under vacuum at 50 °C for 12 hours. Yield: 10.35 g. HPLC analysis: iohexol-O-hexaacetate 5.9 %, gentamycin 0.33 %

### Example 26

### polymerization of MMA to form PMMA particles containing iohexol-O-hexaacetate and clindamycin

A solution of 2.0 % polyvinylalcohol (PVA) (mw 15000) in water (80 ml) containing sodium chloride (20 g) was heated to 80 °C and the solution "bubbled" with argon. A solution of methyl methacrylate (9.0 g), iohexol-O-hexaacetate (Example 1, 1.5 g), clindamycin hydrochloride (1.2 g) and benzoylperoxide (200 mg) was added dropwise to the solution of a period of 30-60 minutes. The mixture was vigorously stirred for 24 hours at 80 °C. The particulate material was isolated by centrifugation (4000 xg for 15 minutes), resuspended in water and isolated by centrifugation. The powder was dried under vacuum at 50 °C for 12 hours. Yield: 10.35 g. HPLC analysis: clindamycin 0.90 %

### Example 27

### Polymerization of MMA to form PMMA particles containing iohexol-O-hexaacetate and clindamycin

A solution of 2.0 % polyvinylalcohol (PVA) (mw 15000) in water (80 ml) with pH adjusted to 10 with sodium hydroxide was heated to 80 °C and the solution "bubbled" with argon. A solution of methyl methacrylate (9.0 g), iohexol-O-hexaacetate (Example 1, 1.5 g), clindamycin hydrochloride (1.2 g) and benzoylperoxide (200 mg) was added dropwise to the solution of a period of 30-60 minutes. The mixture was vigorously stirred for 24 hours at 80 °C. The particulate material was isolated by centrifugation (4000 xg for 15 minutes), resuspended in water and isolated by centrifugation. The powder was dried under vacuum at 50 °C for 12 hours. Yield: 10.35 g. HPLC analysis: clindamycin 0.15 %

### Example 28

### Polymerization of MMA to form very small PMMA particles containing iohexol-O-hexaacetate

A solution of 2.0 % polyvinylalcohol (PVA) (mw 15000) (80 ml) was heated to 80 °C and the solution "bubbled" with argon. A solution of methyl methacrylate (9.0 g), iohexol-O-hexaacetate (Example 1, 1.5 g) and benzoylperoxide (200 mg) was added dropwise to the solution of a period of 30-60 minutes. The mixture was stirred vigorously and treated with a stator-rotor emulsifier (Ultraturax T25, IKA, 20 000 rpm) for 12 hours at 80 C. The PMMA particles were too small that it was not possible to isolate them by normal centrifugation procedure (4000 xg for 15 minutes).

### Example 29

### Particle size of polymethylmethacrylate particles

Particles prepared in Examples 10, 13, 24, 25 and 28 were suspended in water (ultrasound bath for 5 minutes) and examined in a microscope (Leica DMLS). The average size of these particles were compared with average size of commercial Palacos® particles (without zirconium dioxide).

The average size of the particles from Example 13 and 24 were in the same range as for Palacos®.

The average size of the particles from Example 10 and 25 were smaller than for Palacos®.

The particles prepared in Example 28 were very small and the size distribution was in the µm range.

### Example 30

### Release of gentamycin from polymethylmethacrylate particles

Polymethylmethacrylate particles (1.0 g, from example 25) were suspended in saline. The suspension was kept at 37 °C for 10 hours. The particles were centrifugated off and the supernatant was analyzed for gentamycin by HPLC. Gentamycin was qualitatively detected.

### Example 31

### Preparation of bone cement comprising iohexol-hexaacetate in non-homogeneous phase (6.67% in finalbone cement)

Prepolymerized particles of iohexol-containing polymethacrylate (PMMA) (40 g) (from Example 10) were mixed with a solution of methacrylate (18.4 g), N,N-dimethyl-p-toluidine (400 mg), hydroquinone (1.2 mg) and chlorophyll (8.0 mg) under reduced pressure in a syringe. After mixing for 2-3 minutes, the mixture was ready for application.

### Example 32

### Preparation of bone cement comprising iohexol-hexaacetate in homogeneous phase (6.67% in final bone cement)

Prepolymerized particles of polymethylmethacrylate (PMMA) (40 g) (from Example 12) were mixed with a solution of methymethacrylate (14.4 g), iohexol-hexaacetate (4.0 g) (from Example 1), N,N-dimethyl-p-toluidine (400 mg) hydroquinone (1.2 mg) and chlorophyll (8.0 mg) under reduced pressure in a syringe. After mixing for 2-3 minutes, the mixture was ready for application.

### Example 33

### Preparation of bone cement comprising iohexol-hexaacetate homogeneously distributed in the final cement (6.67% in final bone cement)

Prepolymerized particles of iohexol-containing polymethylmethacrylate (PMMA) (40 g) (from Example 11) were mixed with a solution of methymethacrylate (17.1 g), iohexol-hexaacetate (1.33 g) (from Example 1), N,N-dimethyl-p-toluidine (400 mg) hydroquinone (1.2 mg) and chlorophyll (8.0 mg) under reduced pressure in a syringe. After mixing for 2-3 minutes, the mixture was ready for application.

### Example 34

### Preparation of bone cement comprising a polymerizable iodinated compound homogeneously distributed in the final cement

Bone cement is prepared analogously to Example 33 using the PMMA particles of Example 14 and the polymerizable derivative if Example 8 in the MMA homogeneous phase. The concentration of the compound of Example 14 in MMA is selected such that the concentration of iodine is approximately the same throughout the final polymer.

### Example 35

### Preparation of bone cement based on PMMA particles and a polymerizable amide

Bone cement is prepared analogously to Example 32 using the PMMA particles of Example 12 and 5% wt of the polymerizable compound of Example 4.

### Example 36

### Preparation of bone cement comprising iohexol-O-hexaacetate and gentamycin homogeneously distributed in the final cement

Prepolymerized particles of polymethylmethacrylate (PMMA) containing iohexol-O-hexaacetate (Example 1, 5.9 %, 236 mg) and gentamycin sulfate (0.33 %, 13.2 mg) (from Example 25) were mixed with a solution of Palacos® monomer (containing methyl methacrylate and N,N-dimethyl-p-toluidin) (2 ml) added iohexol-O-hexaacetate (5.9 %, 118 mg) and gentamycin sulfate (0.33 %, 6.6 mg). After mixing for 2-3 minutes, the mixture was ready for application.

### Example 37

### Preparation of bone cement comprising iohexol-O-hexaacetate and chloramphenicol homogeneously distributed in the final cement

Prepolymerized particles of polymethylmethacrylate (PMMA) containing iohexol-O-hexaacetate (Example 1, 4.5 %, 180 mg) and chloramphenicol (0.28 %, 11.2 mg) (from Example 23) were mixed with a solution of Palacos® monomer (containing methyl methacrylate and N,N-dimethyl-p-toluidine) (2 ml) added iohexol-O-hexaacetate (4.5 %, 90 mg) and chloramphenicol (0.28 %, 5.6 mg). After mixing for 2-3 minutes, the mixture was ready for application. Example 38

### Mechanical properties of bone cement with iohexol-O-hexaacetate - tensile test

Iohexol-O-hexaacetate (4 g), prepared according to Example 1 was dissolved in Palacos® monomer solution (20 ml). The composition of this monomer solution was: methyl methacrylate (18.4g), N,N-dimethyl-p-toluidine (0.38 g) and chlorophyllin (0.4 mg). Bone cement was made from the above solution and Palacos® powder without zirconium dioxide (36 g). The composition of the powder was: polymethylmethacrylate (35.68 g, benzoyl peroxide (0.32 g) and chlorophyllin (1 mg). The cement was vacuum-mixed in an Optivac mixing system and injected into eight half size ISO 527 moulds and aged in saline at 37 °C for a minimum of two weeks.

Palacos® commercial bone cement with zirconium dioxide (6.13 g) was prepared in a similar way and used as reference sample.

Both cements were tested with regard to tensile properties. This was performed on an Instron 8511 load frame instrument.

The cement with iohexol-O-hexaacetate (monomer-soluble contrast agent) showed significantly higher ultimate tensile strength than commercial Palacos® with zirconium dioxide as contrast agent. The ultimate tensile strength was 48.7 MPa for bone cement with iohexol-O-hexaacetate and 42.5 MPa for commercial Palacos® with zirconium dioxide.

### Example 39

### Mechanical properties of bone cement with iohexol-O-hexaacetate - strain

Iohexol-O-hexaacetate based bone cement and commercial Palacos® bone cement prepared as in Example 38 were tested with regard to strain using an Instron extensometer. The ultimate strain for iohexol-O-hexaacetate cement was significantly higher than for commercial Palacos®. The mean ultimate strength for iohexol-O-hexaacetate cement was 0.030 mm/mm and 0.022 mm/mm for commercial Palaces® with zirconium dioxide.

### Example 40

### Mechanical properties of bone cement with iohexol-O-hexaacetate - compressive strength

Iohexol-O-hexaacetate based bone cement and commercial Palacos® bone cement prepared as in Example 38 were tested with regard to compressive strength using an Instron 8511 instrument. The ultimate compressive strength of both cements were similar (about 100 MPa).

### Example 41

### HPLC analyses of iohexol-O-hexaacetate and antibiotics

The HPLC system consisted of an Agilent 1100 model chromatograph equipped with an G1311A quaternary pump, an G1311A autosampler and an G1315B diode-array UV-Vis detector (Agilent Technologies). The analytical column was a ZORBAX Eclipse XDB-C8, 5 µm, 4.6 x 150 mm operated at ambient temperature (Agilent Technologies) (Method A), SULPELCOSIL LC-18, 3 µm, 4.6 x 360 mm operated at ambient temperature (SULPELCO) (Method B) and ZORBAX Extend-C18, 5 µm, 4.6 x 250 mm operated at ambient temperature (Agilent Technologies) (Method C). Iohexol-O-hexaacetate (IHA, Example 1) standard solutions were prepared by dissolving IHA powder in CH₂Cl₂ to give concentrations ranging from 0.5 mg/ml to 2.0 mg/ml. Standards of amoxicillin and chloramphenicol were prepared by dissolving amoxicillin prehydrate and chloramphenicol powder respectively in CH₂Cl₂ to give concentrations ranging from 0.2 mg/ml to 1.0 mg/ml. The gentamycin standard solutions were prepared by adding 500 µl solutions of gentamycin sulfate in purified water 250 µl of phenylisocyanate (5 mg/ml in acetonitrile) and 250 µl of triethylamine (5 mg/ml in acetonitrile) to give concentrations ranging from 50 µg/ml to 200 µg/ml. This pre-column derivatization was performed at room temperature. The clindamycin standard solution was prepared by dissolving clindamycin hydrochloride in water to give a concentration of 3 mg/ml.

### Method A:

PMMA powder (100 mg) containing iohexol-O-hexaacetate and the different antibiotics norfloxacin, amoxicillin and chloramphenicol (Example 21-23) was added CH₂Cl₂ (10 ml) and stirred for 30 minutes. The different solutions were analysed for content of iohexol-O-hexaacetate and antibiotics by HPLC. The mobile phase was isocratic eluation with acetonitrile and methanol in water (30/30/40, v/v/v) with a flow rate of 1.0 ml/min. The detection wavelength was 254 nm and injection volume of both standard samples and samples was 5 µL.

### Method B:

PMMA powder (1.0 g) containing iohexol-O-hexaacetate and gentamycin (Example 24 and 25) was added CH₂Cl₂ (10 ml) and water (10 ml) and stirred for 30 minutes. 0.5 ml of the aqueous solution was filtered through a WHATMAN 0.2 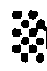 m PTFE w/GMF filter (WHATMAN Inc., Clifton, NJ, USA) and added 250 µl of phenylisocyanate (5 mg/ml in acetonitrile) and triethylamine (5 mg/ml in acetonitrile). The reaction mixture was reacted at room temperature and the resulting solution was shaken several times. The mobile phase was isocratic eluation with acetonitrile in water (40/60, v/v) with a flow rate of 1.5 ml/min. The detection wavelength was 240 nm and injection volume of both standard samples and samples was 10 µL.

**Method C:**

PMMA powder (1.0 g) containing iohexol-O-hexaacetate and clindamycin (Example 26 and 27) was added CH₂Cl₂ (10 ml) and water (10 ml) and stirred for 30 minutes. The aqueous solution was filtered through a WHATMAN 0.2 µm PTFE w/GMF filter (WHATMAN Inc., Clifton, NJ, USA) before injection on the HPLC. The mobile phase was isocratic eluation with acetonitrile and ammonium acetate buffer in water (35/40/25, v/v/v) with a flow rate of 1.5 ml/min. The detection wavelength was 210 nm and injection volume of both standard samples and samples was 5 µL.

**Table 1**

| **Example No.** | **Content of IHA (w/w) (%)** | **Method** | **Content of antibiotic substance (w/w) (%)** | **Method** |
|---|---|---|---|---|
| 21 | 4.3 | A | n.a. | |
| 22 | 2.4 | A | 3.2 | A |
| 23 | 4.5 | A | 0.28 | A |
| 24 | 4.6 | A | Qualitatively detected | B |
| 25 | 5.9 | A | 0.33 | B |
| 26 | n.a. | A | 0.9 | |
| 27 | n.a. | A | 0.15 | |

## Claims

1. A bone cement comprising in admixture a monomer-containing liquid portion and a particulate polymer portion, **characterized in that** at least one of said portions comprises a dissolved non-polymerizable organoiodine compound.

2. A bone cement as claimed in claim 1 additionally comprising an antibiotic compound.

3. A bone cement as claimed in claim 2 wherein said antibiotic compound is selected from gentamicin, colistin, erythromycin, clindamicin, penicillins, norfloxacin and chloramphenicol.

4. A bone cement as claimed in either one of claims 2 and 3 wherein said antibiotic compound is present in the form of a lipophilic ester.

5. A bone cement as claimed in any one of claims 1 to 4 wherein the concentration of the organoiodine compound within the polymer particles portion differs by less than 50% compared to the concentration of the organoiodine within the polymer which is prepared in situ from the monomer during use.

6. A bone cement as claimed in any one of claims 2 to 4 wherein the concentration of the antibiotic compound within the polymer particles portion differs by less than 50% compared to the concentration of the organoiodine within the polymer prepared in situ from the monomer during use.

7. A bone cement as claimed in either of claims 5 and 6 wherein said concentration difference is less than

8. A bone cement as claimed in any one of claims 1 to 7 wherein the liquid monomer portion additionally comprises at least one of hydroquinone, growth hormone, BMP or vitamins.

9. A bone cement as claimed in any one of claims 1 to 8 wherein said liquid monomer phase is present in a range of from 25 to 45% wt. of cement.

10. A bone cement as claimed in any one of claims 1 to 9 wherein said polymer particle phase additionally comprises at least one of hydroquinone, growth hormone, BMP or vitamins.

11. A bone cement as claimed in any one of claims 1 to 10. wherein said polymer particles have a mode particle size of from 1 to 200 µm.

12. A bone cement as claimed in any one of claims 1 to 11 wherein said polymer particles are polydisperse.

13. A bone cement kit comprising a monomer-containing liquid portion and separate therefrom a particulate polymer portion, wherein at least one of said portions comprises a dissolved non-polymerizable organoiodine compound, said kit optionally and preferably further comprising instructions for the preparation of a bone cement therewith.

## Patentansprüche

1. Knochenklebstoff bzw. -zement, der in Mischung einen monomerhaltigen Flüssigkeitsanteil und einen Polymerpartikelanteil aufweist, **dadurch gekennzeichnet, dass** mindestens einer der Anteile eine gelöste nichtpolymerisierbare Organojod-Verbindung aufweist.

2. Knochenklebstoff nach Anspruch 1, der zusätzlich eine antibiotische Verbindung aufweist.

3. Knochenklebstoff nach Anspruch 2, wobei die antibiotische Verbindung aus Gentamicin, Colistin bzw. Polymyxin, Erythromycin, Clindamycin, Penicillinen, Norfloxacin und Chloramphenicol ausgewählt ist.

4. Knochenklebstoff nach einem von der Ansprüche 2 oder 3, wobei die antibiotische Verbindung in Form eines lipophilen Esters vorliegt.

5. Knochenklebstoff nach einem der Ansprüche 1 bis 4, wobei die Konzentration der Organojod-Verbindung in dem Polymerpartikelanteil sich um weniger als 50 %, verglichen mit der Konzentration des Organojods in dem Polymer, das in situ aus dem Monomer während der Verwendung hergestellt wird, unterscheidet.

6. Knochenklebstoff nach einem der Ansprüche 2 bis 4, wobei die Konzentration der antibiotischen Verbindung in dem Polymerpartikelanteil sich um weniger als 50 %, verglichen mit der Konzentration des Organojods in dem Polymer, das in situ aus dem Monomer während der Verwendung hergestellt wird, unterscheidet.

7. Knochenklebstoff nach einem der Ansprüche 5 oder 6, wobei der Konzentrationsunterschied weniger als 10 % beträgt.

8. Knochenklebstoff nach einem der Ansprüche 1 bis 7, wobei der flüssige Monomeranteil zusätzlich mindestens eine der Komponenten Hydroquinon, Wachstumshormon, BMP oder Vitamine aufweist.

9. Knochenklebstoff nach einem der Ansprüche 1 bis 8, wobei die flüssige Monomerphase in einem Bereich von 25 bis 45 % des Gewichtes des Klebstoffes vorliegt.

10. Knochenklebstoff nach einem der Ansprüche 1 bis 9, wobei die Polymerpartikelphase zusätzlich mindestens eine der Komponenten Hydroquin, Wachstumshormon, BMP oder Vitamine aufweist.

11. Knochenklebstoff nach einem der Ansprüche 1 bis 10, wobei die Polymerpartikel eine Mode- bzw. Form-Partikelgröße von 1 bis 200 µm aufweisen.

12. Knochenklebstoff nach einem der Ansprüche 1 bis 11, wobei die Polymerpartikel polydispers sind.

13. Knochenklebstoffausstattung bzw. -kit, die einen monomerhaltigen Flüssigkeitsanteil und einen hierzu separaten Polymerpartikelanteil aufweist, wobei zumindest einer der Anteile eine gelöste nicht polymerisierbare Organojod-Verbindung aufweist, wobei die Ausstattung wahlweise und bevorzugt weitere Anweisungen für die Herstellung eines Knochenklebstoffs damit aufweist.

## Revendications

1. Ciment osseux comportant, en mélange, une partie liquide contenant un monomère et une partie de polymère particulaire, **caractérisé en ce qu'**au moins une desdites parties comporte un composé organo-iodé non polymérisable dissous.

2. Ciment osseux tel que revendiqué dans la revendication 1, comportant de plus un composé antibiotique.

3. Ciment osseux tel que revendiqué dans la revendication 2, dans lequel ledit composé antibiotique est choisi parmi la gentamicine, la colistine, l'érythromycine, la clindamicine, la pénicilline, la norfloxacine et le chloroamphénicol.

4. Ciment osseux tel que revendiqué dans l'une ou l'autre des revendications 2 ou 3, dans lequel ledit composé antibiotique est présent sous la forme d'un ester lipophile.

5. Ciment osseux tel que revendiqué dans l'une quelconque des revendications 1 à 4, dans lequel la concentration du composé organo-iodé à l'intérieur de la partie de particules de polymère diffère de moins de 50 % comparée à la concentration de l'organo-iodé à l'intérieur du polymère qui est préparé in situ à partir du monomère pendant l'utilisation.

6. Ciment osseux tel que revendiqué dans l'une quelconque des revendications 2 à 4, dans lequel la concentration du composé antibiotique à l'intérieur de la partie de particules de polymère diffère de moins de 50 % comparée à la concentration de l'organo-iodé à l'intérieur du polymère préparé in situ à partir du monomère pendant l'utilisation.

7. Ciment osseux tel que revendiqué dans l'une ou l'autre des revendications 5 et 6, dans lequel ladite différence de concentration est inférieure à 10 %.

8. Ciment osseux tel que revendiqué dans l'une quelconque des revendications 1 à 7, dans lequel la partie de monomère liquide comporte de plus au moins un élément parmi l'hydroquinone, une hormone de croissance, la protéine morphogène osseuse (BMP) ou des vitamines.

9. Ciment osseux tel que revendiqué dans l'une quelconque des revendications 1 à 8, dans lequel ladite phase de monomère liquide est présente dans une plage de 25 à 45 % en poids de ciment.

10. Ciment osseux tel que revendiqué dans l'une quelconque des revendications 1 à 9, dans lequel ladite phase de particules de polymère comporte de plus au moins un élément parmi l'hydroquinone, une hormone de croissance, la protéine morphogène osseuse (BMP) ou des vitamines.

11. Ciment osseux tel que revendiqué dans l'une quelconque des revendications 1 à 10, dans lequel lesdites particules de polymère ont une taille modale de particule de 1 à 200 µm.

12. Ciment osseux tel que revendiqué dans l'une quelconque des revendications 1 à 11, dans lequel lesdites particules de polymère sont polydispersées.

13. Kit de ciment osseux comportant une partie liquide contenant un monomère et, séparée de celle-ci, une partie de polymère particulaire, dans lequel au moins une desdites parties comporte un composé organo-iodé non polymérisable dissous, ledit kit comportant facultativement et de préférence également des instructions pour la préparation d'un ciment osseux avec celui-ci.
